# DEMANDE DE BREVET EUROPEEN

(11) **EP 4 484 934 A1**
(43) Date de publication de la demande: **01.01.2025**
(21) Numéro de dépôt: 24184521.3
(22) Date de dépôt: 26.06.2024
(51) Int. Cl.: G01N 21/64

(54) **DISPOSITIF DE DÉTECTION D'UNE LIAISON ENTRE DEUX ESPÈCES BIOLOGIQUES**

(30) Priorité: 27.06.2023 FR 2306749
(71) Demandeur: Commissariat à l'Energie Atomique et aux Energies Alternatives, 75015 Paris (FR)
(72) Inventeur: CONSTANTIN, Olivier, 38054 GRENOBLE CEDEX 09 (FR); CAILLAT, Patrice, 38054 GRENOBLE cedex 09 (FR)
(74) Mandataire: INNOV-GROUP

(57) **Abrégé**

L'invention concerne un dispositif de caractérisation de l'affinité d'une liaison entre une espèce biologique d'intérêt, par exemple un anticorps, avec une espèce biologique de capture, par exemple un antigène. La détection de la liaison est réalisée par une variation d'une lumière de fluorescence, en utilisant un dispositif de détection comportant des nanofils (30), chaque nanofil agissant comme un transducteur d'une lumière de fluorescence en un signal électrique.

## Description

### DOMAINE TECHNIQUE

Le domaine technique de l'invention est la détection d'une liaison entre deux espèces biologiques par transduction optique effectuée par des nanofils.

### ART ANTERIEUR

Un hybridome est une cellule résultant de l'hybridation artificielle de cellules lymphocytes de mammifères et de cellules de myélomes immortalisées. Les hybridomes sont utilisés pour la production d'anticorps monoclonaux. Ces derniers peuvent être utilisés dans la prévention ou le diagnostic ou le traitement des maladies. Il s'agit de cellules productrices d'anticorps (ASC antibody secreting cells).

La caractérisation d'hybridomes est un processus long et coûteux, visant à sélectionner les hybridomes produisant les anticorps monoclonaux (Mab : Monoclonal antibody) présentant une affinité élevée avec un antigène cible prédéterminé. Cela implique une qualification d'anticorps monoclonaux, et plus précisément une évaluation de leur affinité avec l'antigène cible.

A l'heure actuelle, la qualification d'anticorps monoclonaux est effectuée en utilisant des microbalances à quartz ou des capteurs SPR (Surface Plasmon Résonance ou résonance à plasmons de surface). Ces systèmes comportent une surface de détection fonctionnalisée par une espèce biologique cible, en général un antigène. La formation de liaisons anticorps-antigène modifie la fréquence de résonance des balances à Quartz ou bien la phase ou l'amplitude d un faisceau réfléchi sur la surface des capteurs SPR. Ainsi, le suivi temporel de ces variables permet une évaluation de la constante d'affinité anticorps-antigène. Cependant, il s'agit d'une mesure globale, sans information sur la dispersion de l'affinité anticorps-antigène. De plus, le recours à une microbalance à quartz ou un capteur SPR suppose une grande quantité d'anticorps.

Compte tenu du rendement faible des hybridomes (rendements d'hybridation, et de production), cela implique la caractérisation d'un grand nombre d'hybridomes, afin d'identifier, parmi ces derniers, ceux présentant un bon rendement productif d'un anticorps présentant une bonne affinité avec un antigène cible.

Il est intéressant de pouvoir disposer des solutions de criblage nécessitant une quantifié moindre d'anticorps, de façon à limiter le besoin de culture d'hybridomes, et plus généralement d'ASC.

US2012113419 décrit un dispositif comportant des nanofils pour une application de spectrométrie Raman SERS (Surface Enhanced Raman Spectroscopy). La publication Spies Maria et al « Nanowire photodetectors based on wurtzite semiconductor heterostructures" décrit une matrice de nanofils utilisés dans une cellule photovoltaïque.

US2015/369801 décrit un dispositif permettant de caractériser une affinité entre deux molécules, basé sur la résonance à plasmons de surface.

Les inventeurs proposent un procédé de détermination d'une affinité d'une espèce biologique d'intérêt, en particulier un anticorps monoclonal, avec une espèce biologique de capture, en particulier un antigène, présentant un temps de réponse rapide, compact, et nécessitant une faible quantité de l'espèce biologique d'intérêt.

### EXPOSE DE L'INVENTION

Un premier objet de l'invention est un dispositif de caractérisation d'une affinité de liaison entre une espèce biologique d'intérêt et une espèce biologique de capture, l'espèce biologique d'intérêt et l'espèce biologique de capture étant configurées de façon à induire, sous l'effet de la liaison et d'une exposition à une lumière d'excitation, une variation d'une lumière de fluorescence, dans une bande spectrale de fluorescence, le dispositif comportant :
- un substrat, comportant au moins une première électrode;
- une structure multicouches, comportant au moins une deuxième électrode;
- des nanofils, s'étendant entre la première électrode et la deuxième électrode, parallèlement à un axe transversal;
- une couche d'encapsulation s'étendant autour des nanofils, entre le substrat et la structure multicouches, la couche d'encapsulation étant formée d'un matériau isolant ;
- la structure multicouches comportant :
   - une couche conductrice, comportant la deuxième électrode;
   - une couche d'interface, électriquement isolante, recouvrant la deuxième électrode, la deuxième électrode étant interposée entre la couche d'interface et un nanofil, la couche d'interface étant configurée pour être disposée entre un échantillon, comportant l'espèce biologique d'intérêt, et la deuxième électrode, la couche d'interface étant délimitée par une surface de fonctionnalisation, fonctionnalisée par l'espèce biologique de capture, de telle sorte que la surface de fonctionnalisation forme une interface entre le dispositif et l'échantillon;
   - la structure multicouche étant telle que la ou chaque deuxième électrode et la couche d'interface sont transparentes dans la bande spectrale de fluorescence ;
le dispositif étant tel que :
- chaque nanofil comporte une jonction de type homojonction, ou une hétérojonction, ou une jonction Schottky entre la première électrode et la deuxième électrode ;
- la première électrode et la deuxième électrode sont configurées pour être raccordées à un circuit de détection;
- de telle sorte que chaque nanofil forme un nanophotodétecteur dans la bande spectrale de fluorescence, la lumière de fluorescence détectée par chaque nanofil induisant un signal électrique de détection dans le circuit de détection ;
le dispositif comportant une unité de traitement, programmée pour :
- acquérir le signal de détection;
- déterminer une variation, dans le temps, d'une lumière de fluorescence
- en fonction de la variation, détermination d'une caractéristique représentative d'une affinité entre l'espèce biologique d'intérêt et l'espèce biologique de capture.

Le dispositif peut comporter une chambre fluidique, en contact avec la surface de fonctionnalisation.

Selon une possibilité, la surface de fonctionnalisation est segmentée en différents sites de capture, chaque site de capture comportant l'espèce biologique de capture, chaque site de capture étant configuré pour former une liaison avec l'espèce biologique d'intérêt.

Selon une possibilité, la chambre fluidique comporte des canaux, chaque canal s'étendant face à plusieurs sites de capture.

Selon une possibilité, chaque nanofil comporte :
- une homojonction de type pn ;
- ou une hétérojonction ;
- ou une jonction Schottky de type p-métal ou n-métal.

Selon une possibilité, :
- la couche d'interface comporte deux sous-couches, empilées l'une sur l'autre, formant une sous-couche inférieure et une sous-couche supérieure, la sous-couche inférieure étant interposée entre la couche conductrice et la sous-couche supérieure ;
- la sous-couche supérieure comporte des puits, débouchant dans la sous-couche inférieure, chaque puits étant disposé face à un nanofil, chaque puits formant une partie de la surface de fonctionnalisation ;
- la surface de fonctionnalisation est segmentée au niveau de chaque puits, de façon que chaque puits forme un site de capture.

Selon une possibilité, le dispositif comporte plusieurs nanofils, s'étendant entre une même première électrode et une même deuxième électrode, les nanofils formant une grappe de nanofils.

Selon une possibilité, le dispositif comporte plusieurs grappes de nanofils, distantes les unes des autres, de telle sorte qu'un nanofil d'une grappe est plus proche d'un autre nanofil de ladite grappe que d'un autre nanofil d'une autre grappe, les nanofils d'une même grappe s'étendant entre une même première électrode et une même deuxième électrode.

Selon une possibilité, le dispositif comporte plusieurs nanofils, le dispositif étant tel que :
- plusieurs premières électrodes sont formées sur le substrat, et plusieurs deuxièmes électrodes sont formées sur la structure multicouche, chaque nanofil s'étendant entre une première électrode et une deuxième électrode ;
- chaque première électrode est reliée à une première unité d'adressage, configurée pour sélectionner au moins une première électrode ;
- chaque deuxième électrode est reliée à une deuxième unité d'adressage, configurée pour sélectionner au moins une deuxième électrode ;
- de telle sorte que le circuit de détection détecte un courant de détection induit par chaque nanofil s'étendant entre la première électrode et la deuxième électrode sélectionnées.

Un deuxième objet de l'invention est un procédé de caractérisation d'une affinité entre une espèce biologique d'intérêt avec une espèce biologique de capture, à l'aide d'un dispositif selon le premier objet de l'invention, le procédé comportant :
a) disposition d'un échantillon liquide, comportant l'espèce biologique d'intérêt, au contact de la surface de fonctionnalisation ;
b) établissement d'une liaison entre l'espèce biologique d'intérêt et l'espèce biologique de capture, portée par la surface de fonctionnalisation, sur un site de capture situé à l'aplomb d'au moins un nanofil ;
c) excitation du site de capture par une lumière d'excitation, de façon à entraîner une variation d'une lumière de fluorescence émise au niveau du site du capture ;
d) formation d'un signal de détection, par le circuit de détection, le signal de détection étant représentatif de la variation de la fluorescence ;
e) en fonction du signal de détection, détermination d'une caractéristique de l'affinité entre l'espèce biologique d'intérêt et l'espèce biologique de capture.

Selon une possibilité :
- les étapes c) et d) sont réitérées ;
- lors de l'étape e), la caractéristique de l'affinité entre l'espèce biologique d'intérêt et l'espèce biologique de capture est déterminée en fonction du signal de détection formé lors de plusieurs itérations de l'étape d).

Selon une possibilité :
- la liaison entre une espèce biologique d'intérêt et l'espèce biologique de capture entraîne une augmentation ou une diminution de la lumière de fluorescence ;
- sous l'effet de l'augmentation ou de la diminution de la lumière de fluorescence, le signal de détection varie entre un premier niveau et un deuxième niveau ;
- l'étape e) comporte une détermination d'une durée durant laquelle le signal de détection correspond au deuxième niveau, en supposant que sur le site de capture, l'augmentation ou la diminution de la lumière de fluorescence est due à une liaison d'une seule espèce biologique d'intérêt sur l'espèce biologique de capture.

Selon une possibilité:
- les liaisons entre une pluralité d'espèces biologiques d'intérêt identiques et des espèces biologiques de capture entraîne une augmentation ou une diminution de la lumière de fluorescence ;
- suite à des premières itérations des étapes c) à d), les étapes c) et d) sont réitérées lors de deuxièmes itération ;
- préalablement aux deuxièmes itérations des étapes c) et d), un échantillon liquide, considéré comme sans l'espèce biologique d'intérêt, est disposé au contact de la surface de fonctionnalisation ;
- lors de l'étape e), la caractéristique de l'affinité entre l'espèce biologique d'intérêt et l'espèce biologique de capture est déterminée en fonction de signaux de détection formés lors des premières et deuxièmes itérations des étapes c) et d).

Selon une possibilité :
- l'espèce biologique d'intérêt est liée à un marqueur fluorescent ;
- la liaison avec l'espèce biologique de capture entraîne une augmentation de la lumière de fluorescence.

Selon une possibilité :
- l'espèce biologique de capture est liée à un marqueur fluorescent ;
- l'espèce biologique d'intérêt est liée à un extincteur de la lumière de fluorescence ;
- la liaison avec l'espèce biologique de capture entraîne une diminution de la lumière de fluorescence.

L'espèce biologique d'intérêt peut être une protéine et l'espèce biologique de capture peut être une anti-protéine. L'espèce biologique d'intérêt peut être un anticorps et l'espèce biologique de capture peut être un antigène.

Les caractéristiques des revendications dépendantes seront ajoutées ici avant le dépôt. L'invention sera mieux comprise à la lecture de l'exposé des exemples de réalisation présentés, dans la suite de la description, en lien avec les figures listées ci-dessous.

### FIGURES

Les figures 1A et 1B montrent les principaux composants d'un dispositif selon l'invention.
La figure 1C montre une distribution spatiale de sites de capture.
La figure 1D représente un exemple de dispositif selon l'invention, dans lequel les électrodes sont réparties selon un agencement matriciel.
La figure 1E montre un exemple de mise en oeuvre du dispositif.
La figure 1F schématise une évolution, en fonction du temps, d'un signal de détection, selon un premier mode de réalisation de l'invention.
La figure 1G schématise une évolution, en fonction du temps, d'un signal de détection, selon un deuxième mode de réalisation de l'invention
La figure 2A montre une configuration selon laquelle l'espèce biologique d'intérêt est marquée par un fluorophore, ce qui correspond à l'exemple représenté sur la figure 1E.
La figure 2B montre une configuration selon laquelle l'espèce biologique d'intérêt est marquée par un extincteur de fluorescence (quencher), et l'espèce biologique de capture est marquée par un fluorophore.
La figure 2C montre une configuration selon laquelle l'espèce biologique d'intérêt est marquée par un premier fluorophore, et l'espèce biologique de capture est marquée par un deuxième fluorophore. La bande spectrale d'excitation du deuxième fluorophore correspond à la bande spectrale de fluorescence du premier fluorophore.
Les figures 3A à 3E schématisent des étapes de fabrication de nanofils selon un procédé dit ascendant.
La figure 4 représentent différents agencements possibles des nanofils.
Les figures 5A et 5B montrent deux structures différentes de nanofils.
La figure 6 montre différentes étapes de mise en oeuvre du procédé.

### EXPOSE DE MODES DE REALISATION PARTICULIERS

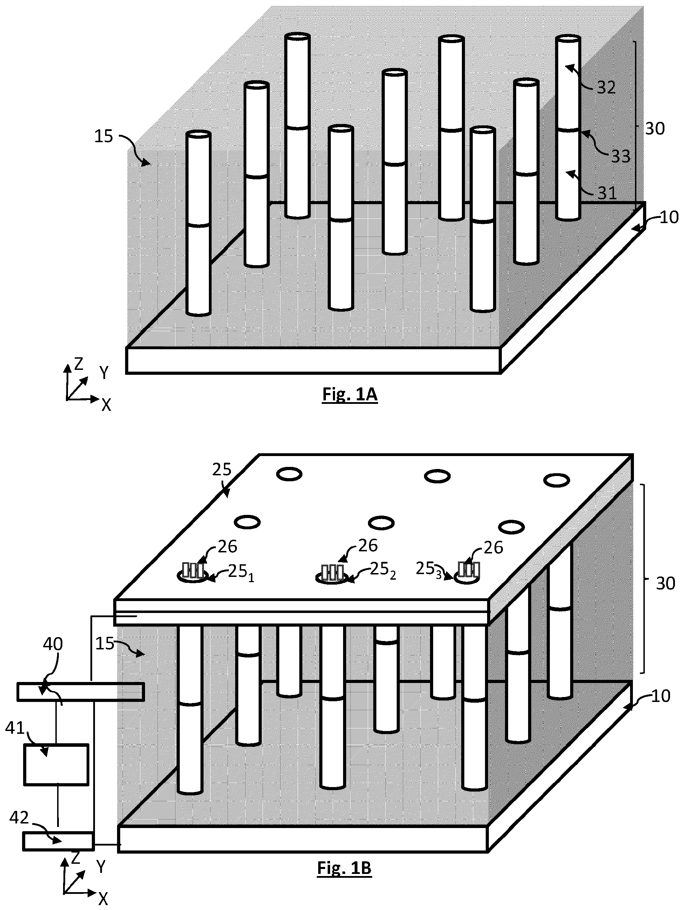

On a représenté, sur les figures 1A à 1E, un premier exemple de dispositif d'analyse 1 permettant une mise en oeuvre de l'invention. Le dispositif d'analyse 1 est configuré pour être placé au contact d'un échantillon 2, comportant par exemple un milieu liquide susceptible de contenir une espèce biologique d'intérêt 28, dont on souhaite caractériser l'affinité avec une espèce biologique de capture 26, pouvant également être désignée espèce biologique cible. Dans cet exemple, les espèces biologiques d'intérêt sont des anticorps monoclonaux et les espèces biologiques de capture (ou espèces biologiques cibles) sont des antigènes. Le dispositif est destiné à caractériser l'affinité de la liaison de l'anticorps monoclonal sur l'antigène formant l'espèce biologique de capture.

Le dispositif comporte un substrat 10, formant ou comportant au moins une première électrode 11_{c}. Dans l'exemple représenté sur la figure 1A, le substrat est un substrat de silicium cristallin, par exemple un substrat Si d'orientation cristalline (111). Le substrat 10 est délimité par une surface, dite première surface 11, comportant une première électrode 11_{c}. Dans l'exemple de la figure 1A, la première surface 11 est formée de Si comportant des régions conductrices. Selon une autre possibilité le substrat 10 fait l'objet d'un dépôt d'une couche conductrice, par exemple du graphène, formant tout ou partie de la première surface 11.

Des nanofils 30 sont formés sur le substrat 10, et plus précisément à partir de la première surface 11. La première surface 11 s'étend selon un plan P_{XY}. Le plan P_{XY} est défini par un axe longitudinal X et un axe latéral Y. Les axes X et Y sont sécants, et de préférence perpendiculaires l'un par rapport à l'autre. Les nanofils s'étendent parallèlement à un axe transversal Z sécant du plan P_{XY} . Dans les modes de réalisation décrits par la suite, l'axe transversal Z est perpendiculaire du plan P_{XY}. La première surface 11 est conductrice au moins au niveau de l'intersection avec chaque nanofil 30. La totalité de la première surface 11 peut être conductrice.

Selon d'autres configurations, les nanofils peuvent être inclinés et non perpendiculaires au plan P_{XY}. Par exemple, si l'orientation cristalline du matériau formant le substrat 10 est (001), les nanofils peuvent croître selon une direction (111), donc en biais par rapport au plan P_{XY}.

Les nanofils 30 ont de préférence un diamètre compris entre 1 nm et 500 nm et une hauteur, selon l'axe transversal Z, comprise entre 300 et 1000 nm, voire 10000 nm.

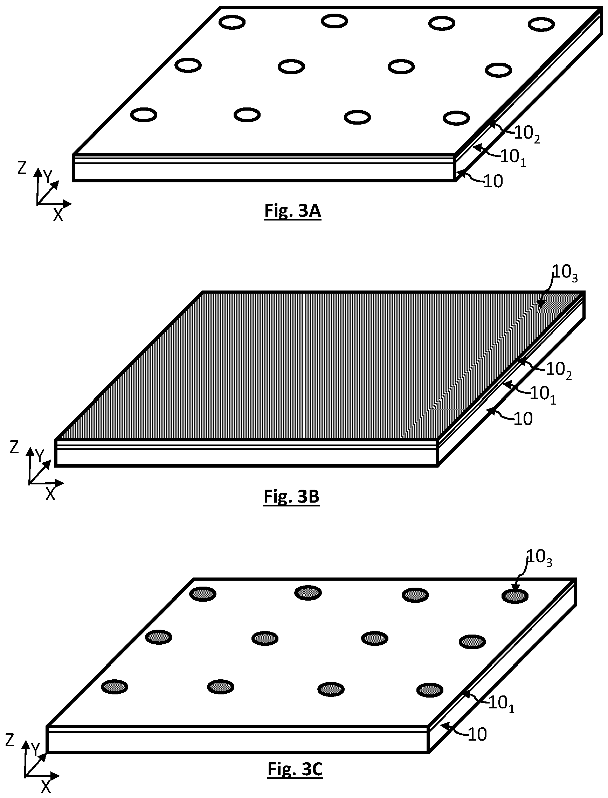

Les nanofils 30 peuvent être synthétisés directement sur le substrat 10, comme décrit en lien avec les figures 3A à 3E. Les nanofils peuvent être formés sur un autre substrat, puis transférés sur le substrat 10. Le transfert peut être effectué comme décrit dans la publication Valente, et al., « Light-Emitting GaAs Nanowires on a Flexible Substrate », Nano Lett 2018, 18(7) 4206-4213.

Les nanofils 30 s'étendent, à partir de la première surface 11, jusqu'à une deuxième surface 21 délimitant une structure multicouches 20. De même que la première surface 11, la deuxième surface 21 est conductrice au moins au niveau de l'intersection avec chaque nanofil 30. Dans l'exemple représenté sur la figure 1B, la deuxième surface 21 est formée à partir d'une couche 22 d'un matériau conducteur transparent dans une bande spectrale de détection décrite ci-après. Il peut par exemple s'agir d'ITO (oxyde d'indium étain).

Chaque nanofil est formé d'un ou plusieurs matériaux semi-conducteurs, et éventuellement d'un matériau métallique. Chaque nanofil comporte une jonction 33. Dans l'exemple représenté, la jonction 33 est une homojonction : chaque nanofil comporte une première partie 31, adjacente de la première surface 11, et une deuxième partie 32, adjacente de la deuxième surface 21. Les première et deuxième parties sont formées d'un même matériau semi-conducteur, avec respectivement deux dopages différents : ainsi, la première partie 31 et la deuxième partie 32 sont respectivement formés d'un même matériau semi-conducteur respectivement dopé n et p ou p et n. Dans l'exemple représenté, la première partie 31 est formée de GaAs (arséniure de gallium) dopé p et la deuxième partie est formée de GaAs dopé n. L'interface entre les deux parties forme la jonction pn 33.

De façon alternative, la jonction 33 peut être effectuée au niveau d'un métal disposé sur ou au contact de la surface 21 de façon à établir une jonction semi-conducteur - métal de type Schottky.

Chaque nanofil comporte au moins un semi-conducteur choisi parmi les matériaux des colonnes III et V, usuellement désignés par le terme matériaux III-V, par exemple, GaAs. Il peut de préférence s'agir d'un matériau de la colonne III et d'arsenic, par exemple InAs (arséniure d'indium). Dans l'exemple représenté, les nanofils 30 sont formés de GaAs. D'autres matériaux semi-conducteurs peuvent être envisagés, par exemple, et de façon non limitative, Si, InGaAs, AlGaAs, InGaP, InGaN, GaN, ZnSe, ZnS, ZnO, ZnCdO, ZnTe, CdSe, Ge, GeSn .

Entre la première surface 11 et la deuxième surface 21, les nanofils 30 sont noyés dans une couche d'encapsulation 15 formée d'un matériau isolant. La couche d'encapsulation 15 peut être formée d'un matériau de type PMMA (Polyméthacrylate de méthyle), BCB (Benzocyclobuthène) ou matériaux SOG (Spin-On Glass) constitués principalement d'oxydes de silicium et d'autres additifs chimiques pour conférer des propriétés spécifiques telles que l'adhérence et la stabilité thermique. La couche d'encapsulation 15 peut être déposée par spin-coating (dépôt à la tournette).

La couche d'encapsulation 15 est de préférence formée suite à la croissance des nanofils, préalablement au dépôt d'une deuxième couche conductrice 22, délimitée par la deuxième surface 21, et destinée à former des deuxièmes électrodes 21_{c}. Les deuxièmes électrodes 21_{c} sont formées, au niveau de la deuxième surface 21, à partir de la couche conductrice 22. La couche conductrice 22 peut être structurée de façon qu'au niveau de la deuxième surface 21, plusieurs deuxièmes électrodes 21_{c} soient électriquement isolées les unes des autres. Ainsi, la couche conductrice peut comporter des ouvertures ou des matériaux isolant délimitant les électrodes 21_{c}. Cela permet une détection différenciée au niveau de chaque nanofil.

Outre la couche conductrice 22, la structure multicouches 20 comporte une couche d'interface 23, adjacente de la couche conductrice 22. La couche d'interface 23 est transparente dans la bande spectrale de détection. La couche d'interface 23 peut par exemple être formée d'une couche d'un polymère, par exemple le PMMA (Polyméthylméthacrylate). La couche d'interface 23 est électriquement isolante, en particulier au niveau de l'interface avec la couche conductrice 22. La couche d'interface 23 est de préférence plane.

La couche d'interface 23 est destinée à former une interface entre la couche conductrice 22, formant les électrodes 21_{c}, et l'échantillon 2. Ainsi, la couche d'interface 23 s'étend entre la couche conductrice 22 et l'échantillon 2. Un aspect important du dispositif est que l'échantillon n'est pas en contact direct avec les nanofils. Il est isolé de ces derniers par la couche d'interface 23.

La couche d'interface 23 est par exemple une couche mince formée de SiO₂ ou de PMMA. La surface de la couche d'interface 23, destinée à être en contact avec l'échantillon est une surface, dite surface de fonctionnalisation 25, fonctionnalisée par des sondes biologiques de capture 26. Il est important que la couche d'interface 23 soit formée d'un matériau présentant une auto-fluorescence aussi faible que possible dans la bande spectrale de détection.

La surface de fonctionnalisation 25, qui forme une interface entre la structure multicouches 20 et l'échantillon 2 à analyser, est fonctionnalisée par des espèces de capture 26.

La couche d'interface 23 peut être nanostructurée, de façon que des nanopuits 27 soient formés à l'interface entre la structure multicouches 20 et l'échantillon à analyser. Le diamètre ou la plus grande diagonale des nanopuits peut être comprise entre 70 nm et 700 nm. Les nanopuits 27 peuvent par exemple être agencés de façon matricielle, ou, de façon plus générale, selon un motif prédéterminé. La surface de fonctionnalisation est alors fonctionnalisée au niveau de chaque nanopuits 27, les espaces entre chaque puits n'étant pas fonctionnalisés. La formation des nanopuits peut être obtenue par amincissement local de la couche d'interface 23.

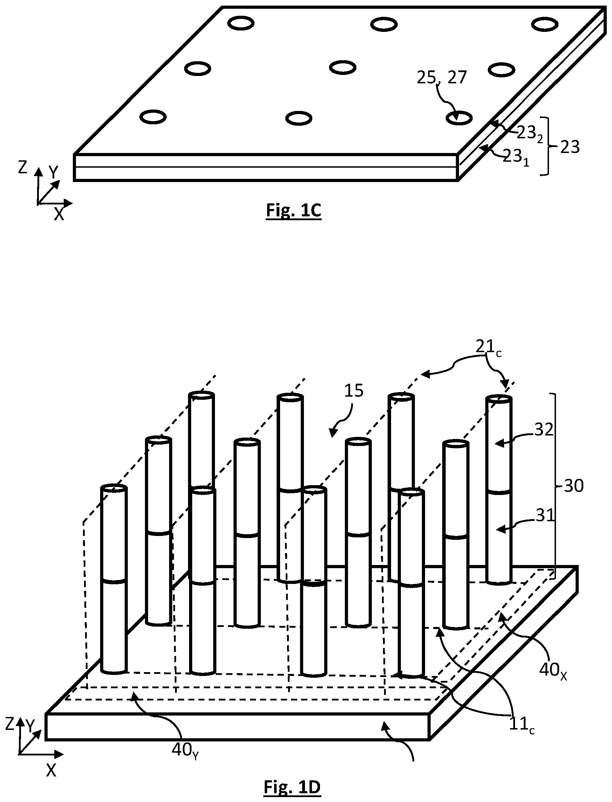

Selon une possibilité, représentée sur la figure 1C, la couche d'interface 23 comporte deux sous-couches superposées 23₁ et 23₂. La couche d'interface comporte une sous-couche inférieure 23₁ interposée entre la couche conductrice 22 et une sous-couche supérieure 23₂. Les nanopuits sont formés par amincissement local de la sous-couche supérieure 23₂, de façon que les nanopuits débouchent dans la sous-couche inférieure 23₁. La sous-couche supérieure 23₂ peut être formée d'un matériau non fonctionnalisable, par exemple un matériau anti-biofouling (anti-encrassement biologique), par exemple un matériau hydrophobe. Une telle structuration permet que la fonctionnalisation de la surface de fonctionnalisation 25 soit effectuée uniquement au niveau des nanopuits 27, sur la sous-couche inférieure 23₁. Chaque nanopuits 27 forme ainsi un site de capture de l'espèce biologique d'intérêt 28.

De façon plus générale, la surface de fonctionnalisation 25 peut être fonctionnalisée selon un motif de fonctionnalisation prédéterminé. En dehors du motif de fonctionnalisation, la surface de fonctionnalisation n'est pas fonctionnalisée

La fonctionnalisation peut être effectuée par un traitement de la surface de fonctionnalisation 25, par exemple un traitement de surface plasma/oxygène. Lorsque la couche d'interface 23 est formée de PMMA, un traitement plasma/oxygène permet de former des fonctions carboxyles. Les espèces de capture 26 peuvent être greffées sur la surface de fonctionnalisation par liaison covalente.

Lorsque la fonctionnalisation de la surface de fonctionnalisation 25 est effectuée selon un motif spatial, les parties de la surface de fonctionnalisation en dehors du motif spatial peuvent être revêtues d'un revêtement anti-biofouling (anti-encrassement biologique) de façon à éviter une adsorption non spécifique de l'espèce biologique d'intérêt. Un exemple de matériau anti-biofouling est un polyéthylène glycol ou un polymère fluoré.

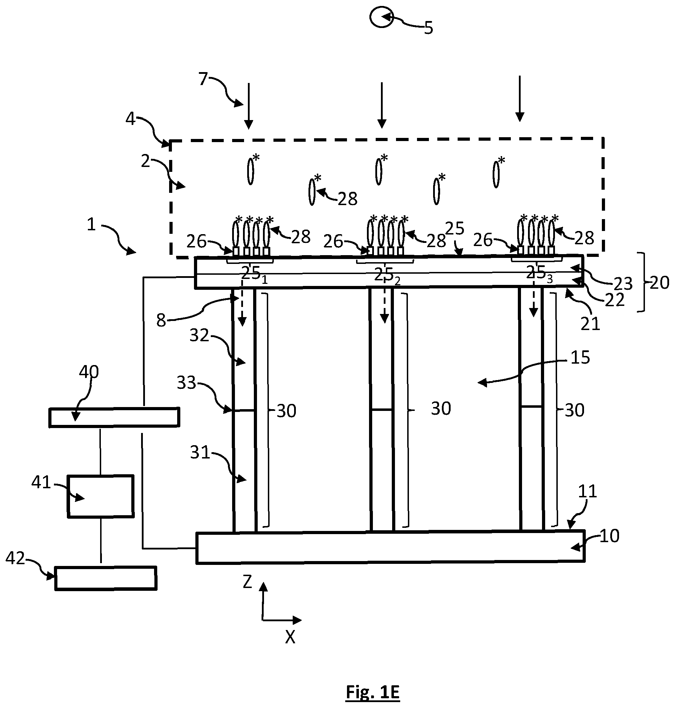

Comme représenté sur les figures 1B et 1E, le dispositif comporte un circuit de détection 40, dont une première borne est raccordée à une première électrode 11_{c}, sur le substrat 10, et une deuxième borne est raccordée à une deuxième électrode 21_{c}, sur la structure multicouches 20. Le circuit de détection 40 permet de mesurer la différence de potentiel, ou un courant électrique, entre la première électrode 11_{c} et la deuxième électrode 21_{c}.

Comme décrit en lien avec les figures 1A à 1C, chaque nanofil s'étend entre une première extrémité, sur une première surface 11 du substrat 10 et une deuxième extrémité, sur une deuxième surface 21 de la structure multicouche 20. La première surface 11 et la deuxième surface 21 sont conductrices au moins au niveau de chaque intersection avec un nanofil. Ainsi, au niveau de chaque intersection avec un nanofil, la première surface comporte une première électrode 11_{c} et la deuxième surface comporte une deuxième électrode 21_{c}. Dans l'exemple représenté sur les figures 1A à 1B, la première surface 11 et la deuxième surface 21 sont formées d'un matériau conducteur. Elles sont conductrices selon toute leur aire.

Les première et deuxième surfaces peuvent être structurées, et comporter différentes électrodes 11_{c}, 21_{c} isolées les unes des autres. Sur la figure 1D, chaque électrode est matérialisée par une ligne en pointillés. Sur la première surface 11, chaque première électrode 11_{c} décrit une ligne, parallèle à l'axe longitudinal X. Sur la deuxième surface 21, chaque deuxième électrode 21_{c} décrit une colonne, parallèle à l'axe latéral Y. Chaque nanofil est fonctionnel lorsque les électrodes 11_{c}, 21_{c}, entre lesquelles il s'étend, sont polarisées. La structuration des électrodes en lignes / colonnes permet de sélectionner les nanofils fonctionnels, ces derniers s'étendant entre deux électrodes polarisées : selon cet agencement, les nanofils reliés à une ligne et à une colonne polarisées sont fonctionnels. Par nanofil fonctionnel, on entend un nanofil polarisé pour effectuer une détection de photons de fluorescence.

Plusieurs lignes et/ou plusieurs colonnes peuvent être polarisées simultanément ou successivement. Le circuit de détection 40 comporte :
- une unité d'adressage 40_{X}, destinée à polariser tout ou partie des premières électrodes 11_{c}, parallèles à l'axe X,
- et une unité d'adressage 40_{Y} destinée à polariser tout ou partie des deuxièmes électrodes 21_{c} parallèles à l'axe Y.

Les parties fonctionnalisées de la surface de fonctionnalisation forment des sites de capture de l'espèce biologique d'intérêt.

Comme précédemment indiqué, chaque espèce biologique de capture 26 est configurée pour capturer une espèce biologique d'intérêt, par exemple un anticorps. Par capture, on entend l'établissement d'une liaison entre l'espèce biologique de capture 26 et l'espèce biologique d'intérêt 28.

Sur la figure 1E, on a représenté une chambre fluidique 4, dans laquelle on a disposé un échantillon liquide 2 comportant des espèces biologique d'intérêt 28. La chambre fluidique est disposée en dessus de la surface de fonctionnalisation, de façon que la surface de fonctionnalisation forme une paroi de la chambre fluidique. La chambre fluidique 4 peut comporter des canaux, de façons à diriger l'échantillon face à tout ou partie des sites de capture, selon un cheminement prédéfini.

Sur la surface de fonctionnalisation, on a représenté trois sites de capture 25₁, 25₂ et 25₃. Au niveau de chaque site de capture, la surface de fonctionnalisation 25 est fonctionnalisée. Dans cet exemple, les anticorps monoclonaux, formant les espèces biologiques d'intérêt 28, sont fonctionnalisés par un marqueur fluorescent symbolisé par le symbole *. Chaque site de capture est fonctionnalisé par un même antigène 26.

La source de lumière est configurée pour émettre une lumière d'excitation dans une durée courte, de l'ordre de quelques ps à une ou quelques ns. Il peut par exemple s'agir d'une source de lumière laser. Cela permet de générer, au niveau de chaque cluster, un signal de fluorescence, qui dépend de la quantité d'anticorps 28 liés aux antigènes 26.

Un aspect important de l'invention, décrit par la suite, consiste à traduire le signal de fluorescence en un signal électrique dépendant du marqueur fluorescent, ce qui permet d'obtenir, en chaque site de capture, un signal électrique dépendant de la quantité d'anticorps liés à l'antigène 26.

L'invention tire profit de la capacité d'un nanofil à détecter un signal optique, dans une bande spectrale prédéfinie, et à convertir le signal optique en un signal électrique de détection. Ainsi, le dispositif 1 est basé sur une détection optique de liaisons antigène-anticorps induisant une réponse électrique du dispositif.

La source de lumière 5 génère une lumière d'excitation 7, qui se propage à travers l'échantillon 2 jusqu'à la surface de fonctionnalisation 25. Sous l'effet de l'illumination à la longueur d'onde d'excitation une lumière de fluorescence 8 est émise par le marqueur fluorescent *, dans une longueur d'onde de fluorescence, plus élevée que la longueur d'onde d'excitation. Des photons de fluorescence sont émis, formant la lumière de fluorescence 8. Du fait de la transparence de la couche d'interface 23, ou du matériau 22 constituant l'électrode 21_{c}, certains photons de fluorescence se propagent à travers un nanofil 30. Lorsqu'un photon de fluorescence est absorbé au niveau de la jonction 33, des paires électrons/trous sont formées. Sous l'effet de la différence de potentiel entre la première partie 31 et la deuxième partie 32, les électrons se propagent à travers la portion dopée n, vers le potentiel le plus élevé tandis que les trous se propagent en sens inverse à travers la portion dopée p. Il en résulte une augmentation du courant électrique circulant dans le circuit de détection 40, ce qui se traduit par une augmentation du signal de détection.

Le choix du matériau semi-conducteur formant le nanofil 30 dépend de la bande spectrale d'absorption dudit matériau, cette dernière devant à la fois contenir la longueur d'onde de fluorescence, et, de préférence, ne pas contenir la longueur d'onde d'excitation du marqueur fluorescent. Le nanofil agit ainsi en tant que nanophotodétecteur de la lumière de fluorescence, tout en n'étant pas sensible à la longueur d'onde d'excitation. Chaque nanofil forme ainsi un filtre vis-à-vis de la longueur d'onde d'excitation. Eventuellement, une couche absorbante peut être ajoutée entre la surface de fonctionnalisation 25 et la couche conductrice 22 pour réduire la largeur spectrale de la réponse des nanofils, par example une couche d'oxide de zinc déposée par pulvérisation. Lorsque le matériau semi-conducteur est GaAs, le marqueur fluorescent peut être Cy3 (Cyanine) : longueur d'onde d'excitation 540nm et longueur d'onde d'émission comprise entre 555 et 600nm.

De préférence, le diamètre des nanofils est contrôlé de façon à conférer une sensibilité dans une bande spectrale étroite. Par bande spectrale étroite, on entend une largeur de bande typiquement de quelques dizaines de nm, et de préférence inférieure à 100 nm. La largeur de bande correspond à la largeur à mi-hauteur du pic d'absorption sur le spectre d'absorption. La largeur de bande de détection est par exemple de l'ordre de 50 nm. Il est ainsi possible d'ajuster la bande spectrale de détection, de façon qu'elle comprenne la longueur d'onde de fluorescence du marqueur fluorescent et qu'elle ne comprenne pas la longueur d'onde d'excitation du marqueur fluorescent. La faculté de nanofils à former un photodétecteur sélectif en longueur d'onde a été décrite dans Mokkapati et al., « Optical design of nanowire absorbers for wavelength selective photodetectors », Sci. Rep. 2015, 5 15339.

La sensibilité spectrale de détection peut être ajustée par l'incorporation de puits quantiques ou de boites quantiques (quantum dots) au niveau de la jonction 33. Cela permet d'obtenir une jonction 33 dont la composition est différente par rapport au reste du nanofil. La longueur d'onde détectée correspond alors à la longueur d'onde du gap défini par le puits ou la boîte quantique.

Chaque nanofil forme ainsi un nanophotodétecteur.

Dans cet exemple, la liaison antigène-anticorps se traduit par une variation de la lumière de fluorescence. En l'occurrence, il s'agit d'une augmentation de la lumière de fluorescence. De préférence, la concentration d'anticorps, dans la chambre fluidique, est ajustée de façon à greffer un seul ou un faible nombre d'anticorps, typiquement entre moins de vingt, à l'aplomb de chaque nanofil.

La lumière de fluorescence détectée par le nanofil disposé à l'aplomb d'un site de capture passe d'un premier niveau, en l'absence d'anticorps greffés, à un deuxième niveau, représentatif de la quantité d'anticorps capturés.

On désigne par S un signal de détection, résultant du circuit de détection 40, formé à partir des charges collectées au niveau de chaque nanofil. Le dispositif comporte une unité de traitement 41, permettant une caractérisation du signal de détection. L'unité de traitement 41 peut comporter un microprocesseur, relié à une mémoire 42 dans laquelle des instructions sont mémorisées. L'unité de traitement 41 peut être programmée pour déterminer une durée durant laquelle le signal de détection occupe un niveau haut.

Le dispositif peut comporter un site de capture non fonctionnalisé, ce qui permet d'établir le premier niveau du signal de détection, en l'absence de liaison anticorps -antigène.

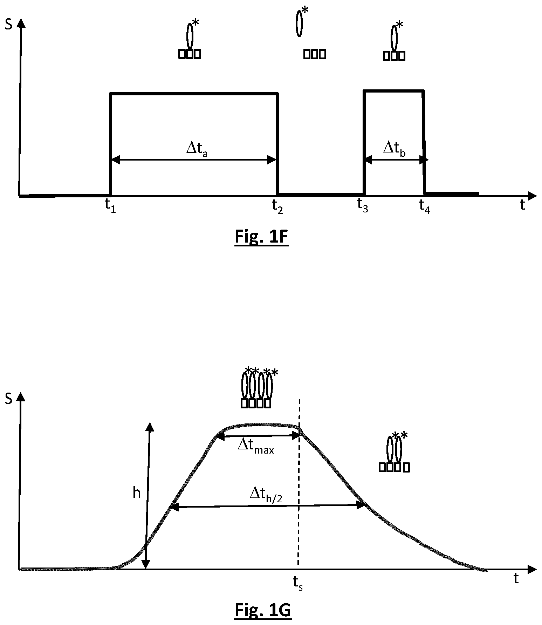

Le dispositif permet de caractériser l'affinité de l'anticorps vis-à-vis d'un antigène. Lorsque la concentration d'anticorps est suffisamment faible, on peut effectuer une détection d'une liaison unique anticorps-antigène à la surface d'un nanofil. Cela correspond à un premier mode de réalisation de l'invention, décrit en lien avec la figure 1F. Selon ce mode de réalisation, on peut détecter chaque greffage et en déterminer la durée. Le fait de disposer de plusieurs sites de capture permet d'obtenir une statistique de mesure.

La concentration d'anticorps et/ou d'antigène est alors déterminée en prenant en compte le nombre de nanofils, la surface 25 et le volume de la chambre fluidique. En supposant que la hauteur de la chambre fluidique est de 100 µm, et que la surface 25 mesure 1 cm², avec un nanofil répartis de façon matricielle selon un pas spatial de 1 µm, soit 10⁸ nanofils, la concentration d'anticorps est d'au moins 1 ng/ml.

Sur la figure 1F,on a schématisé une évolution d'un signal de détection (axe des ordonnées) en fonction du temps (axe des abscisses). Le signal de détection traduit l'intensité de la lumière de fluorescence détectée par un nanofil. A un instant t₁, un anticorps est lié à un antigène situé sur un site de capture, à l'aplomb d'un nanofil. La liaison s'étend selon une durée Δtₐ, jusqu'à un instant t₂. A un instant t₃, postérieur à t₂, un anticorps est lié à un antigène situé sur le même site de capture. La liaison s'étend selon une durée Δt_{b}, jusqu'à un instant t₄. Entre les instants t₂ et t₃, aucun anticorps n'est relié à un antigène au niveau du site de capture.

Selon ce mode de réalisation, on peut déterminer une durée moyenne de chaque liaison anticorps-antigène à l'aplomb d'un même nanofil. Le grand nombre de nanofils permet d'obtenir une statistique de mesure correcte pour caractériser l'affinité par une durée moyenne d'accroche entre les anticorps et les antigènes.

Le mode de réalisation décrit en lien avec la figure 1F est particulièrement intéressant, car il perme une caractérisation individuelle de l'affinité des anticorps, par une mesure de la durée de greffage anticorps-antigène. Il ne suppose pas que la concentration des anticorps soit connue, ni la densité surfacique de l'espèce biologique de capture.

Selon un deuxième mode de réalisation, plusieurs anticorps identiques sont greffés sur un même site de capture, à l'aplomb d'un nanofil, ou de plusieurs nanofils disposés à l'aplomb d'un même site de capture. Dans ce cas, le dispositif permet d'établir des caractéristiques de l'affinité de l'anticorps vis-à-vis de l'antigène.

Sur la figure 1G,on a schématisé une évolution d'un signal de détection (axe des ordonnées) en fonction du temps (axe des abscisses). Sous l'effet du greffage progressif d'anticorps sur le site de capture, l'intensité du signal de fluorescence augmente, jusqu'à atteindre un plateau. Sur le plateau, certains anticorps se détachent ensuite, et sont remplacés par d'autres. On atteint ainsi un équilibre entre l'association des anticorps et la dissociation des anticorps. A un instant tₛ, la chambre fluidique 4 est alimentée par un tampon, sans anticorps. Les anticorps se détachent peu à peu du site de capture, ce qui entraîne une décroissance du signal de fluorescence. L'affinité peut être caractérisée par un ratio entre une durée du plateau (Δtmax) et une durée correspondant à une demi-hauteur du plateau (Δt_{h/2}). h correspond à la hauteur du plateau. Il est possible de calculer les pentes montantes et descendantes et de calculer leur ratio.

Quel que soit le mode de réalisation, on détermine une caractéristique de l'affinité en excitant chaque nanofil à une fréquence d'excitation prédéterminée, et en analysant le signal de détection obtenu suite à chaque excitation. Sur chaque site de capture, le signal de détection est acquis durant une période temporelle de détection postérieure à l'excitation. Cela permet de déterminer un nombre de sites de capture sur lesquels au moins une liaison antigène-anticorps a été produite.

Le fait de prévoir différents sites de capture, mettant en oeuvre un même couple antigène-anticorps, permet d'établir une statistique relativement à chaque site : cela permet de prendre en compte une variabilité de la liaison antigène-anticorps. Les signaux de détection résultant des différents sites de capture sont acquis séquentiellement, en jouant sur l'adressage des électrodes.

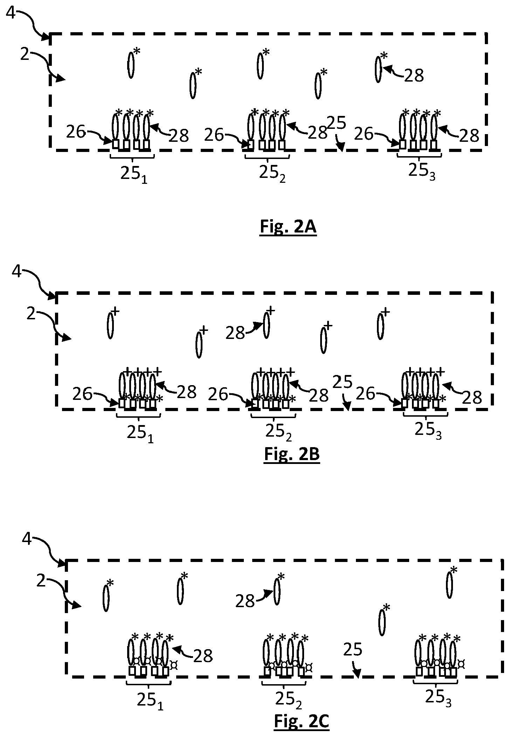

La figure 2A correspond à la configuration décrite en lien avec la figure 1E. Le marquage de l'anticorps peut être réalisé via un anticorps secondaire.

Sur la figure 2B, on a représenté une configuration selon laquelle l'espèce biologique de capture 26 est marqué par un marqueur fluorescent, matérialisé par le symbole *. L'espèce biologique d'intérêt 28, en l'occurrence l'anticorps 28, est lié à un quencher (désactivateur de fluorescence) matérialisé par le symbole +. Le quencher induit une réduction ou une extinction d'une émission des photons de fluorescence. En l'absence de capture, des photons de fluorescence sont émis, ce qui entraîne une circulation d'un courant électrique dans le circuit électrique de l'unité de détection 40. Suite à une capture, l'émission des photons de fluorescence est réduite, ou stoppée, par le quencher ce qui entraîne une variation du courant électrique. Selon ce mode de réalisation, sous l'effet de l'établissement de liaisons anticorps-antigène, le courant de détection diminue.

Sur la figure 2C, on a représenté une configuration selon laquelle l'espèce biologique de capture 26 est marquée par un premier fluorophore, matérialisé par le symbole ¤, et l'espèce biologique d'intérêt est marquée par un deuxième fluorophore, matérialisé par le symbole *. Le premier fluorophore ¤ émet une lumière de fluorescence dans une première bande spectrale de fluorescence sous l'effet d'une illumination dans une première bande spectrale d'excitation. Le deuxième fluorophore * émet une lumière de fluorescence dans une deuxième bande spectrale de fluorescence sous l'effet d'une illumination dans une deuxième bande spectrale d'excitation. La deuxième bande spectrale d'excitation correspond à la première bande spectrale de fluorescence. Lorsque le premier fluorophore et le deuxième fluorophore sont à proximité l'un de l'autre, par effet FRET (Fôrster Résonance Energy Transfert), sous l'effet d'une illumination dans la première bande spectrale d'excitation, une lumière de fluorescence est émise dans la deuxième bande spectrale de fluorescence.

Ainsi, selon ce mode de réalisation, lors d'une illumination dans la première bande spectrale d'excitation :
- en l'absence de liaison antigène-anticorps, une lumière de fluorescence est émise, au niveau de la surface de fonctionnalisation 25, dans la première bande spectrale de fluorescence ;
- en présence d'une liaison antigène-anticorps, une lumière de fluorescence est émise, au niveau de la surface de fonctionnalisation 25, dans la deuxième bande spectrale de fluorescence ;

De préférence, la bande spectrale de détection du nanofil comporte la deuxième bande spectrale de fluorescence et ne comporte pas la première bande spectrale de fluorescence. Ainsi, le nanofil est aveugle dans la première bande spectrale de fluorescence. En l'absence de liaison antigène-anticorps, aucun signal de fluorescence n'est détecté. L'établissement d'une liaison antigène-anticorps entraîne la détection de la lumière de fluorescence, ce qui entraîne une augmentation du signal de détection.

Le recours à des nanofils est particulièrement avantageux. Cela permet d'obtenir un signal exploitable en utilisant un faible nombre d'anticorps monoclonaux.

La bande spectrale d'excitation est de préférence comprise entre 350nm et 550 nm. La bande spectrale de détection de chaque nanofil est de préférence dimensionnée de façon à permettre une détection de la lumière de fluorescence tout en masquant la lumière d'excitation. De façon alternative, les courants de détection, respectivement formés au niveau de chaque nanofil durant l'excitation et l'émission de la lumière de fluorescence, peuvent être temporellement séparés. On tire profit du temps de réponse rapide des nanofils, de l'ordre de la ps, comme décrit dans la littérature Gallo, et al., « Picosecond response times in GaAs/AlGaAs core/shell nanowire-based photodetectors » Appl. Phys. Lett. 2011, 98(24) 241113.

On va à présent décrire différents aspects de conception du dispositif selon l'invention.

### Formation des nanofils

Les figures 3A à 3E schématisent des étapes de formation de nanofils à partir d'un substrat 10 de silicium. Les figures 3A à 3E correspondent à une approche dite montante (ou bottom-up). Le substrat 10 est formé de Si, d'orientation (111) et comporte une couche superficielle 10₁ de SiO₂, destinée à former une couche barrière, d'épaisseur 10nm - 15 nm. La couche de SiO₂ est recouverte d'une couche 10₂ de PMMA d'épaisseur 45 nm. Les couches 10₁ et 10₂ font l'objet d'une gravure, de façon à former des nanopuits isolés les uns des autres, selon un motif prédéterminé. Cf. figure 3A. Les nanopuits débouchent sur le substrat de Si. Une fine couche de métal 10₃, par exemple de l'or, d'épaisseur 5 à 10 nm est déposée sur la couche de SiO₂. Le métal ajouté, en l'occurrence l'or, joue le rôle de catalyseur. Cf. figure 3B. L'excès d'or entre les nanopuits est éliminé par lift of (retrait) de la couche 10₂ de PMMA. Cf. figure 3C. On obtient ainsi des ilots d'or 10₃ isolés les uns des autres, aux positions correspondant à la position des nanopuits préalablement formés.

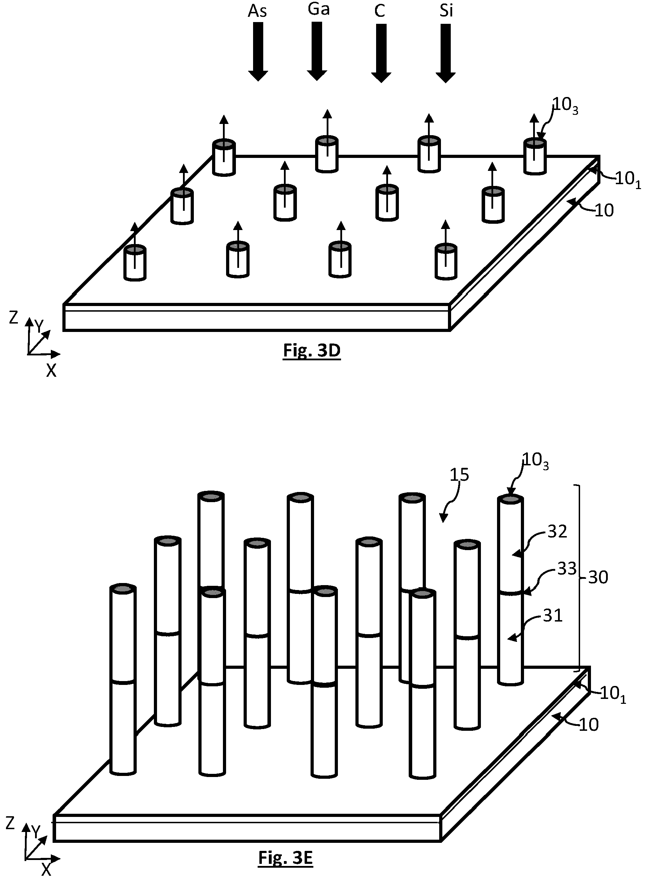

Après chauffage à une température supérieure à 450°C, les ilots forment des gouttes. Les gouttes d'or jouent le rôle de catalyseur. Les nanofils de semi-conducteur sont ensuite formés par épitaxie par jet moléculaire (MBE : molecular beam epitaxy). Cela consiste à envoyer un ou plusieurs jets moléculaires vers le substrat pour réaliser une croissance épitaxiale. Cf. figure 3D. Les jets moléculaire en phase vapeur comportent les espèces chimiques composant le nanofil semi-conducteur ainsi que les espèces dopantes. Les espèces moléculaires heurtent et diffusent dans les gouttelettes d'or. Lorsque ces dernières atteignent une saturation, la nucléation des nanofils se produit tout d'abord aux interfaces gouttelettes/substrat, puis aux interfaces gouttelettes/nanofils en formation.

Le procédé n'est pas limité à l'utilisation d'or en tant que catalyseur. D'autres catalyseurs peuvent être utilisés, par exemple Ga. Lorsque le catalyseur est Ga et que Ga constitue également un élément constituant le semi-conducteur, on parle de croissance de nanofils autocatalysée.

Ce procédé permet par exemple une croissance de nanofils en utilisant As, Ga ainsi que C et Si respectivement comme dopant p et n, la température de mise en oeuvre étant de 610°C. Le procédé se poursuit jusqu'à ce que les nanofils atteignent une hauteur prédéterminée. Cf. figure 3E.

Suite à l'étape représentée sur la figure 3E, la couche d'encapsulation 15 est formée entre les nanofils, par exemple par spin-coating. La couche d'encapsulation permet d'isoler électriquement les nanofils les uns des autres, et confère une meilleure tenue mécanique à l'ensemble. Une gravure plasma et/ou un pollissage peut être effectuée au niveau de l'extrémité des nanofils opposée au substrat, de façon à éliminer les résidus du catalyseur et homogénéiser la hauteur des nanofils et de la couche d'encapsulation 15.

La couche conductrice 22, puis la couche d'interface 23, sont ensuite successivement déposées sur l'ensemble formé par les nanofils et la couche d'encapsulation 15.

Dans le mode de réalisation décrit sur les figures 3A à 3E, la formation des nanopuits sur le substrat 10 permet de maîtriser la position des nanofils.

Un autre avantage de l'approche montante, décrite en lien avec les figures 3A à 3E est qu'elle permet un contrôle plus précis de la structure cristalline des nanofils. L'approche montante permet une incorporation maîtrisée de boites quantiques (quantum dots) ou de puits quantiques, en contrôlant leur position, en particulier selon l'axe Z.

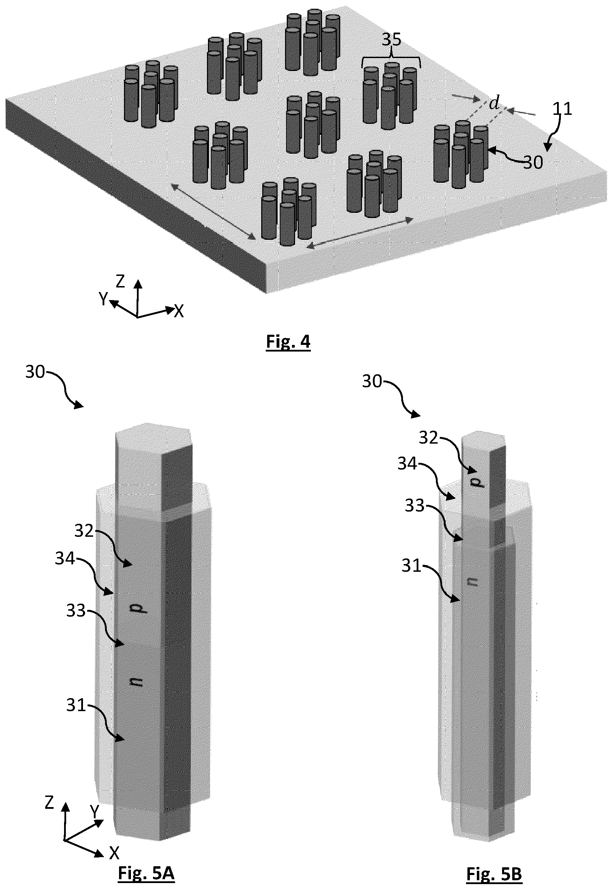

La figure 4 illustre une configuration selon laquelle les nanofils sont agencés en formant des grappes 35, ou clusters. Les nanofils d'un même cluster sont rapprochés les uns des autres, la distance d entre deux nanofils adjacents d'une même grappe étant de préférence supérieure ou égale au diamètre des nanofils. La distance entre deux grappes adjacentes peut être égale ou supérieure à deux fois la distance d

Lorsque les nanofils sont répartis en clusters, comme décrit en lien avec la figure 4, les nanofils d'un même cluster sont de préférence raccordés à une même électrode, à la fois sur le substrat 10 et sur la structure multicouche 20. Les nanofils d'un même cluster sont ainsi simultanément fonctionnels. Les nanofils non reliés au circuit de détection ne sont pas fonctionnels.

L'agencement des nanofils en clusters 35 peut être combiné avec une structuration de la surface de fonctionnalisation en nanopuits 27, comme décrit en lien avec la figure 1B. Dans ce cas, chaque nanopuits 27 s'étend face aux nanofils appartenant à un même cluster 35.

La disposition des nanofils selon un agencement prédéfini sur le substrat 10 n'est pas nécessaire. Selon une possibilité, des gouttes de catalyseur métallique sont réparties de façon aléatoire sur le substrat 10. Suite à l'adjonction, en phase vapeur, des molécules constituant le nanofil, par exemple Ga, As et des dopants, par exemple C, Si, les nanofils se développent à partir des positions initialement occupées, sur le substrat, par les gouttes de catalyseur.

Selon une autre possibilité, les nanofils sont obtenus par gravure, selon une approche dite descendante (ou top down). L'approche descendante est décrite dans la demande de brevet FR2114563 déposée le 27/12/2021.

### Fonctionnalisation - fluorescence

La fonctionnalisation de la surface de fonctionnalisation 25 est assurée par le greffage de sondes biologiques de capture 26 sur la surface de fonctionnalisation 25. Les sondes biologiques de capture 26 sont destinées à capturer sélectivement une espèce biologique d'intérêt, dont on souhaite caractériser l'affinité avec la sonde biologique de capture.

Selon un mode de réalisation, en multiplexage, différentes sondes biologiques de capture 26, sont séparées sur la surface de fonctionnalisation 25. En référence avec la figure 4, la partie de la surface de fonctionnalisation située en vis-à-vis d'une même grappe 35 peut être fonctionnalisée par une même sonde biologique de capture 26, de façon à adresser une même espèce biologique d'intérêt. On tire alors profit de la détection potentielle, par plusieurs nanofils adjacents, de la fluorescence résultant d'une liaison entre l'espèce biologique de capture et l'espèce biologique d'intérêt. Cela permet de caractériser l'affinité entre l'espèce biologique de capture et l'espèce biologique d'intérêt à partir de plusieurs sites de capture, et cela de façon séquentielle.

Deux sites de capture différents 25₁ 25₂ de la surface de fonctionnalisation 25, respectivement disposées en vis-à-vis de deux grappes différents, peuvent être fonctionnalisées par deux espèces biologiques de capture différentes, de façon à adresser différents couples espèce biologique de capture / espèce biologique d'intérêt.

La chambre fluidique 4 peut comporter des canaux, de façons à diriger simultanément un même anticorps vers différentes lignes, chaque ligne étant formée de sites de capture fonctionnalisés avec la même espèce biologique d'intérêt. Deux canaux différents sont isolés les uns de l'autre de façon à pouvoir tester l'affinité d'un même anticorps à différents sites de capture respectivement fonctionnalisés par différentes espèces de capture.

Du fait de la sensibilité élevée de chaque nanofil, le nombre de nanofils composant une même grappe peut être relativement faible. Ainsi, la surface, dans le plan P_{XY}, de chaque grappe, est faible, chaque grappe adressant sélectivement un couple espèce biologique de capture / espèce biologique d'intérêt. Il est ainsi possible de disposer un grand nombre de grappes, adressant respectivement des couples identiques, ou des couples différents, dans un même dispositif compact. Il en résulte le recours à un volume réduit de réactifs. La sensibilité des nanofils permet de détecter caractériser des anticorps peu affins.

Un autre avantage de l'invention est que l'affinité peut être caractérisée par des mesures temporelles, indépendamment de l'intensité maximale du signal de détection. Elle ne suppose pas une connaissance de la densité des antigènes ou de la concentration des anticorps dans la chambre fluidique.

### Structure des nanofils

Les figures 5A et 5B représentent d'autres structures de nanofils pouvant être mises en oeuvre dans un dispositif selon l'invention. La figure 5A représente un nanofil similaire aux nanofils précédemment décrits. La jonction 33 est disposée axialement, en s'étendant entre deux parties 31, 32, de dopage différents, espacées l'une de l'autre selon l'axe transversal Z. Dans l'exemple de la figure 5A, une gaine de passivation 34 contourne le nanofil.

Dans l'exemple de la figure 5B, la jonction 33 s'étend radialement entre deux zones de dopage différents. Ainsi, la jonction 33 s'étend autour de l'axe transversal Z, parallèlement à ce dernier. La première partie 31 et la deuxième partie 32 sont séparées radialement, la séparation entre les deux parties correspondant à un rayon de séparation. La première partie s'étend entre l'axe du nanofil et la jonction 33, tandis que la deuxième partie s'étend autour de la jonction 33.

Une structure axiale, est considérée comme avantageuse car elle favorise une incorporation de puits quantiques ou de points quantiques à l'intérieur des nanofils afin d'ajuster le spectre d'absorption.

Une structure radiale permet de disposer d'une jonction 33 s'étendant selon une hauteur importante selon l'axe Z, ce qui permet d'augmenter la sensibilité de détection. De façon optionnelle, la structure radiale représentée sur la figure 5B comporte une gaine annulaire 34 telle que décrite en lien avec la figure 5A.

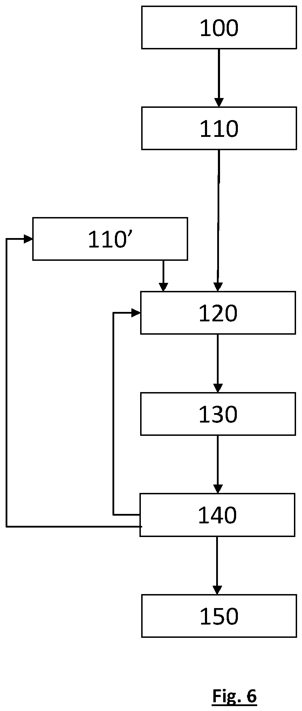

La figure 6 schématise les principales étapes d'un procédé de mise en oeuvre de l'invention.

Etape 100 : disposition de la chambre microfluidique sur la surface de fonctionnalisation, cette dernière ayant été fonctionnalisée, de façon à obtenir des sites de capture, chaque site de capture étant fonctionnalisé par un antigène.

Etape 110 : écoulement de l'échantillon, comportant les anticorps, dans la chambre fluidique.

Etape 120 : excitation d'un ou plusieurs sites de capture par une lumière d'excitation.

Etape 130 : suite à chaque excitation, détection d'une variation de la lumière de fluorescence.

Etape 140 : acquisition d'un signal de détection, représentatif de la variation de la lumière de fluorescence au niveau de chaque site de capture.

Etape 150 : en fonction du signal de détection, détermination d'une caractéristique de l'affinité entre au moins un antigène, greffé au niveau d'un site de capture, et un anticorps contenu dans l'échantillon.

Les étapes 120 et 140 sont réitérées, de façon à obtenir une pluralité de signaux de détection en fonction du temps. L'étape 150 est mise en oeuvre à partir des signaux de détection respectivement acquis au cours de chaque étape 140.

Selon une possibilité, décrite en lien avec la figure 1F, suite à des premières itérations des étapes 120 à 140, le procédé comporte une étape 110', au cours de laquelle un échantillon, considéré comme dépourvu d'espèce biologique d'intérêt, s'écoule dans la chambre fluidique 4. Les étapes 120 à 140 font l'objet de deuxièmes itérations. L'étape 150 est mise en oeuvre à partir des signaux de détection respectivement acquis au cours des premières et deuxièmes itérations des étapes 120 à 140.

Le dispositif tire profit d'un temps de réponse rapide, typiquement de l'ordre de la ns. On observe que le dispositif ne nécessite pas le recours à des composants optiques volumineux. De plus, la réponse du dispositif est stable, et peu sensibles aux variations environnementales : pH de l'échantillon, température, présence de molécules ou d'ions différents de la biomolécule d'intérêt. Cela est dû au fait que les nanofils ne sont pas au contact de l'échantillon, mais isolés, physiquement et électriquement, de ce dernier par la couche d'interface 23.

Enfin, le dispositif étant basé sur des nanophotodétecteurs, il permet d'obtenir une plateforme d'analyse compacte. Le dispositif peut être obtenu en mettant en oeuvre un procédé de fabrication collective, ce qui permet d'abaisser le coût.

De plus, le dispositif est sensible, ce qui permet d'être utilisé pour caractériser l'affinité d'un faible nombre d'anticorps monoclonaux. Cela limite le besoin de cultiver et cloner un grand nombre d'hybridomes, et cela permet de diminuer la durée nécessaire pour déterminer la qualité de production d'une ASC. Les anticorps peuvent être prélevés dans le surnageant d'un milieu de culture comportant au moins une cellule de type ASC. L'objectif est d'identifier rapidement les hybridomes, et de façon plus générale les ASC, produisant les anticorps présentant une forte affinité avec des antigènes prédéterminés. L'invention permet une mise en oeuvre d'un criblage fonctionnel nécessitant une faible quantité d'anticorps.

L'invention peut également s'appliquer à la caractérisation d'anticorps générés in-silico, par exemple sur la base d'algorithmes mettant en oeuvre l'intelligence artificielle.

Bien que décrite en lien avec une détermination de l'affinité anticorps-antigène, l'invention peut s'appliquer, plus généralement, pour caractériser une affinité d'interactions de type protéine - antiprotéine, ou l'affinité de fragments d'anticorps avec des antigènes.

## Revendications

1. Dispositif (1) de caractérisation d'une affinité de liaison entre une espèce biologique d'intérêt (28) et une espèce biologique de capture (26), l'espèce biologique d'intérêt et l'espèce biologique de capture étant configurées de façon à induire, sous l'effet de la liaison et d'une exposition à une lumière d'excitation, une variation d'une lumière de fluorescence, dans une bande spectrale de fluorescence, le dispositif comportant :
- un substrat (10), comportant au moins une première électrode (11_{c}) ;
- une structure multicouches (20), comportant au moins une deuxième électrode (21_{c}) ;
- des nanofils (30), s'étendant entre la première électrode (11_{c}) et la deuxième électrode (21_{c}), parallèlement à un axe transversal (Z) ;
- une couche d'encapsulation (15) s'étendant autour des nanofils, entre le substrat et la structure multicouches (20), la couche d'encapsulation étant formée d'un matériau isolant ;
- un circuit de détection (40) ;
- la structure multicouches comportant :
• une couche conductrice (22), comportant la deuxième électrode;
• une couche d'interface (23), électriquement isolante, recouvrant chaque deuxième électrode (21_{c}), la deuxième électrode étant interposée entre la couche d'interface et un nanofil, la couche d'interface étant configurée pour être disposée entre un échantillon, comportant l'espèce biologique d'intérêt, et la deuxième électrode, la couche d'interface étant délimitée par une surface de fonctionnalisation (25), fonctionnalisée par l'espèce biologique de capture, de telle sorte que la surface de fonctionnalisation forme une interface entre le dispositif et l'échantillon;
• la structure multicouche étant telle que la deuxième électrode et la couche d'interface sont transparentes dans la bande spectrale de fluorescence ;
le dispositif étant tel que :
- chaque nanofil (30) comporte une jonction (33) de type homojonction, ou une hétérojonction, ou une jonction Schottky entre la première électrode et la deuxième électrode ;
- la première électrode et la deuxième électrode sont configurées pour être raccordées au un circuit de détection (40);
- de telle sorte que chaque nanofil forme un nanophotodétecteur dans la bande spectrale de fluorescence, la lumière de fluorescence détectée par chaque nanofil induisant un signal électrique de détection dans le circuit de détection ;
le dispositif comportant une unité de traitement, programmée pour :
• acquérir le signal de détection;
• déterminer une variation, dans le temps, d'une lumière de fluorescence
• en fonction de la variation, détermination d'une caractéristique représentative d'une affinité entre l'espèce biologique d'intérêt et l'espèce biologique de capture.

2. Dispositif selon la revendication 1, comportant une chambre fluidique (4), en contact avec la surface de fonctionnalisation.

3. Dispositif selon l'une quelconque des revendications précédentes, dans lequel la surface de fonctionnalisation (25) est segmentée en différents sites de capture, chaque site de capture comportant l'espèce biologique de capture, chaque site de capture étant configuré pour former une liaison avec l'espèce biologique d'intérêt.

4. Distribution selon l'une quelconque des revendications 2 et 3, dans lequel la chambre fluidique comporte des canaux, chaque canal s'étendant face à plusieurs sites de capture.

5. Dispositif selon l'une quelconque des revendications précédentes dans lequel :
- la couche d'interface comporte deux sous-couches (23₁, 23₂), empilées l'une sur l'autre, formant une sous-couche inférieure et une sous-couche supérieure, la sous-couche inférieure étant interposée entre la couche conductrice (22) et la sous-couche supérieure ;
- la sous-couche supérieure comporte des puits, débouchant dans la sous-couche inférieure, chaque puits étant disposé face à un nanofil, chaque puits formant une partie de la surface de fonctionnalisation ;
- la surface de fonctionnalisation est segmentée au niveau de chaque puits, de façon que chaque puits forme un site de capture.

6. Dispositif selon l'une quelconque des revendications précédentes, comportant plusieurs nanofils, s'étendant entre une même première électrode et une même deuxième électrode, les nanofils formant une grappe de nanofils.

7. Dispositif selon la revendication 6, comportant plusieurs grappes de nanofils (35), distantes les unes des autres, de telle sorte qu'un nanofil d'une grappe est plus proche d'un autre nanofil de ladite grappe que d'un autre nanofil d'une autre grappe, les nanofils d'une même grappe s'étendant entre une même première électrode et une même deuxième électrode.

8. Dispositif selon l'une quelconque des revendications précédentes, comportant plusieurs nanofils, le dispositif étant tel que :
- plusieurs premières électrodes sont formées sur le substrat, et plusieurs deuxièmes électrodes sont formées sur la structure multicouche, chaque nanofil s'étendant entre une première électrode et une deuxième électrode ;
- chaque première électrode est reliée à une première unité d'adressage (40_{X}), configurée pour sélectionner au moins une première électrode ;
- chaque deuxième électrode est reliée à une deuxième unité d'adressage (40_{Y}), configurée pour sélectionner au moins une deuxième électrode ;
- de telle sorte que le circuit de détection (40) détecte un courant de détection induit par chaque nanofil s'étendant entre la première électrode et la deuxième électrode sélectionnées.

9. Procédé de caractérisation d'une affinité entre une espèce biologique d'intérêt (28) et une espèce biologique de capture (26) à l'aide d'un dispositif selon l'une quelconque des revendications précédentes, le procédé comportant :
a) disposition d'un échantillon liquide, comportant l'espèce biologique d'intérêt, au contact de la surface de fonctionnalisation ;
b) établissement d'une liaison entre l'espèce biologique d'intérêt et l'espèce biologique de capture, portée par la surface de fonctionnalisation, sur un site de capture situé à l'aplomb d'au moins un nanofil ;
c) excitation du site de capture par une lumière d'excitation, de façon à entraîner une variation d'une lumière de fluorescence émise au niveau du site du capture ;
d) formation d'un signal de détection, par le circuit de détection, le signal de détection étant représentatif de la variation de la fluorescence ;
e) en fonction du signal de détection, détermination d'une caractéristique de l'affinité entre l'espèce biologique d'intérêt et l'espèce biologique de capture.

10. Procédé selon la revendication 9, dans lequel
- les étapes c) et d) sont réitérées ;
- lors de l'étape e), la caractéristique de l'affinité entre l'espèce biologique d'intérêt et l'espèce biologique de capture est déterminée en fonction du signal de détection formé lors de plusieurs itérations de l'étape d).

11. Procédé selon la revendication 10, dans lequel
- la liaison entre une espèce biologique d'intérêt et l'espèce biologique de capture entraîne une augmentation ou une diminution de la lumière de fluorescence ;
- sous l'effet de l'augmentation ou de la diminution de la lumière de fluorescence, le signal de détection varie entre un premier niveau et un deuxième niveau ;
- l'étape e) comporte une détermination d'une durée durant laquelle le signal de détection correspond au deuxième niveau, en supposant que sur le site de capture, l'augmentation ou la diminution de la lumière de fluorescence est due à une liaison d'une seule espèce biologique d'intérêt sur l'espèce biologique de capture.

12. Procédé selon la revendication 10, dans lequel :
- les liaisons entre une pluralité d'espèces biologiques d'intérêt identiques et des espèces biologiques de capture entraîne une augmentation ou une diminution de la lumière de fluorescence ;
- suite à des premières itérations des étapes c) à d), les étapes c) et d) sont réitérées lors de deuxièmes itérations ;
- préalablement aux deuxièmes itérations des étapes c) et d), un échantillon liquide, considéré comme sans l'espèce biologique d'intérêt, est disposé au contact de la surface de fonctionnalisation ;
- lors de l'étape e), la caractéristique de l'affinité entre l'espèce biologique d'intérêt et l'espèce biologique de capture est déterminée en fonction de signaux de détection formés lors des premières et deuxièmes itérations des étapes c) et d).

13. Procédé selon l'une quelconque des revendications 9 à 12 dans lequel :
- l'espèce biologique d'intérêt est liée à un marqueur fluorescent ;
- la liaison avec l'espèce biologique de capture entraîne une augmentation de la lumière de fluorescence.

14. Procédé selon l'une quelconque des revendications 9 à 13, dans lequel
- l'espèce biologique de capture est liée à un marqueur fluorescent ;
- l'espèce biologique d'intérêt est liée à un extincteur de la lumière de fluorescence ;
- la liaison avec l'espèce biologique de capture entraîne une diminution de la lumière de fluorescence.

15. Procédé selon l'une quelconque des revendications 9 à 14, dans lequel l'espèce biologique d'intérêt est un anticorps et l'espèce biologique de capture est un antigène.
